Europäisches Patentamt

European Patent Office

Office européen des brevets

(19)

(11) **EP 1 374 852 B1**

(12) **FASCICULE DE BREVET EUROPEEN**

(45) Date de publication et mention
de la délivrance du brevet:
**26.10.2005 Bulletin 2005/43**

(51) Int Cl.⁷: **A61K 7/48**, A61K 7/00,
A61K 7/06

(21) Numéro de dépôt: **03291234.7**

(22) Date de dépôt: **23.05.2003**

(54) **Composition cosmétique et/ou dermatologique contenant au moins un actif hydrophile sensible à l'oxydation stabilisé par au moins un polymère ou copolymère d'anhydride maléique**

Kosmetische und/oder dermatologische Zusammensetzung, die mindestens einen oxidationsempfindlichen hydrophilen durch mindestens einem Polymer oder Copolymer stabilisierten Wirkstoff aus Maleinsäureanhydrid enthält

Cosmetic and/or dermatological Composition containing at least one oxidation-sensitive hydrophilic active stabilised by at least one polymer or copolymer of maleic anhydride

(84) Etats contractants désignés:
**AT BE BG CH CY CZ DE DK EE ES FI FR GB GR HU IE IT LI LU MC NL PT RO SE SI SK TR**

(30) Priorité: **20.06.2002 FR 0207638**

(43) Date de publication de la demande:
**02.01.2004 Bulletin 2004/01**

(73) Titulaire: **L'OREAL**
**75008 Paris (FR)**

(72) Inventeur: **Biatry, Bruno**
**94300 Vincennes (FR)**

(74) Mandataire: **Kromer, Christophe**
**L'OREAL - D.I.P.I.**
**25-29 Quai Aulagnier**
**92600 Asnières (FR)**

(56) Documents cités:
**EP-A- 0 282 951     EP-A- 0 380 367**
**EP-A- 0 815 847     EP-A- 1 151 741**
**WO-A-93/22374     FR-A- 2 801 788**
**FR-A- 2 816 316     US-A- 3 531 427**
**US-A- 6 024 942     US-A- 6 103 267**

Remarques:
Le dossier contient des informations techniques présentées postérieurement au dépôt de la demande et ne figurant pas dans le présent fascicule.

EP 1 374 852 B1

**Description**

La présente invention se rapporte à une composition cosmétique et/ou dermatologique à usage topique contenant au moins un actif hydrophile sensible à l'oxydation et au moins un copolymère d'anhydride maléique, dans un milieu physiologiquement acceptable comprenant une phase aqueuse.

Il est connu d'introduire dans des compositions cosmétiques divers actifs destinés à apporter des traitements spécifiques à la peau et/ou aux cheveux. Toutefois, certains de ces actifs présentent l'inconvénient d'être instables en milieu aqueux et de se dégrader facilement au contact de l'eau, en particulier à cause de phénomènes d'oxydation. Ils perdent ainsi rapidement leur activité au cours du temps et cette instabilité va à l'encontre de l'efficacité recherchée.

On cherche ainsi depuis longtemps à formuler l'acide ascorbique ou vitamine C, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter contre le vieillissement de la peau ou prévenir celui-ci. Malheureusement, en raison de sa structure chimique (d'alpha-cétolactone), l'acide ascorbique est très sensible à certains paramètres de l'environnement et notamment aux phénomènes d'oxydation. Il s'ensuit donc une dégradation rapide de l'acide ascorbique formulé en présence de ces paramètres, et plus particulièrement en présence d'oxygène, de lumière, d'ions métalliques, en fonction de la température, ou encore dans certaines conditions de pH (Pharm. Acta. Helv., 1969, 44, 611-667 ; STP Pharma, 1985, 4, 281-286).

Plusieurs solutions ont donc été envisagées dans l'art antérieur pour diminuer et/ou retarder la dégradation de l'acide ascorbique.

Il a ainsi été proposé d'utiliser l'acide ascorbique sous forme de dérivé chimique (ascorbyl phosphate de magnésium, ou esters d'acides gras et d'acide ascorbique), mais la biodisponibilité de ces dérivés est très faible (J. Am. Acad. Dermatol., 1996, 34, 29-33).

L'instabilité de l'acide ascorbique vis à vis de l'oxygène a pu être améliorée en utilisant des conditionnements particuliers comme des bi-compartiments sous atmosphère inerte tels que décrits dans le brevet US-5,935,584, ou bien encore par l'utilisation d'émulsions à deux phases dont l'une est constituée d'une poudre sèche contenant l'acide ascorbique et la seconde d'une phase liquide. Le mélange des deux phases doit s'effectuer au moment de l'utilisation (WO98/43598). Ces solutions présentent des inconvénients au niveau du coût et de la complexité des fabrications ainsi que des contraintes importantes au niveau de l'utilisation.

Une autre solution proposée dans l'art antérieur consiste à utiliser des glycols ou des polyols en forte concentration afin de diminuer la solubilité de l'oxygène dans la formulation, protégeant ainsi l'acide ascorbique (WO-96/24325, EP 0 755 674, US-5,981,578). Les polyols peuvent éventuellement être incorporés dans des liposomes comme décrit dans le brevet US-6,020,367. Mais ces solutions présentent l'inconvénient de conduire à des formulations collantes dont la cosméticité est difficile à améliorer. Par ailleurs la présence d'une forte concentration de ces composés peut provoquer des phénomènes d'irritation.

L'acide ascorbique peut également être formulé dans des milieux anhydres tels que les silicones (US-6,194,452) qui sont capables de créer une barrière anhydre autour de l'acide ascorbique. Un inconvénient majeur de telles solutions résulte du manque de fraîcheur à l'application.

Il subsiste donc le besoin d'une composition utilisable notamment dans le domaine cosmétique, dans laquelle un actif hydrophile et instable en milieu oxydant est stabilisé, qui soit confortable lors de l'application, qui ne provoque aucune irritation de la peau après application, et qui soit compatible avec les contraintes d'une mise en oeuvre industrielle de son procédé de fabrication.

Le but de la présente invention est de proposer une composition contenant un actif sensible à l'oxydation, présentant de bonnes propriétés cosmétiques, tant au niveau du toucher qu'au niveau de la tolérance et dont la conservation dans le temps ne demande pas de précautions particulières.

La Demanderesse a découvert, de manière fortuite, que l'utilisation d'un copolymère d'anhydride maléique et de styrène dans des compositions dont la phase aqueuse renferme un actif sensible à l'oxydation, tel que l'acide ascorbique, permettait d'atteindre le but précité.

A la connaissance de la demanderesse, un tel polymère comportant une unité d'anhydride maléique n'a jamais été associé à des actifs hydrophiles sensibles aux dégradations par oxydation dans le but d'améliorer leur stabilité. Ceci est vrai en particulier dans le cas de l'acide ascorbique.

La présente invention a donc pour objet une composition à usage topique, contenant au moins un actif hydrophile sensible à l'oxydation et au moins un copolymère d'anhydride maléique et de styrène dans un milieu physiologiquement acceptable comprenant une phase aqueuse. Le copolymère est présent en quantité suffisante pour stabiliser ledit actif hydrophile sensible à l'oxydation. De préférence, l'actif sensible à l'oxydation et le copolymère sont tous deux dans la phase aqueuse.

L'invention concerne également l'utilisation d'un copolymère d'anhydride maléique et de styrène pour stabiliser un actif hydrophile sensible à l'oxydation, en particulier en phase aqueuse.

L'utilisation d'un copolymère d'anhydride maléique et de styrène pour stabiliser un actif hydrophile sensible à l'oxydation présente l'avantage de pouvoir stabiliser cet actif dans des compositions possédant un pH compris entre 5 et 7, c'est à dire dans des conditions qui respectent l'équilibre physiologique de la peau dont le pH est voisin de 5,5.

L'utilisation d'un copolymère d'anhydride maléique et de styrène pour stabiliser un actif hydrophile sensible à l'oxydation est particulièrement adaptée pour stabiliser ledit actif à une concentration en solution aqueuse comprise entre 0,5 et 50% en poids, préférentiellement entre 1 et 30 % en poids, et plus particulièrement entre 4 et 20 % en poids.

Selon l'invention, par actif hydrophile on entend un composé ayant une solubilité dans l'eau d'au moins 0,25 % à température ambiante (25°C).

Selon l'invention, par actif hydrophile *sensible à l'oxydation* on entend tout actif d'origine naturelle ou synthétique susceptible de subir une dégradation par un mécanisme d'oxydation. Ce phénomène d'oxydation peut avoir plusieurs causes, en particulier la présence d'oxygène, de lumière, d'ions métalliques, une température élevée, ou encore certaines conditions de pH.

On peut citer à titre d'exemple et de façon non limitative : l'acide ascorbique et ses dérivés tels que ses sels ou ses esters, en particulier le 5,6-di-O-diméthylsilylascorbate (vendu par la Sté Exsymol sous la référence PRO-AA), le sel de potassuim du dl-alpha-tocopheryl-dl-ascorbyl-phosphate (vendu par la Sté Senju Pharmaceutical sous la référence SEPIVITAL EPC), l'ascorbyl phosphate de magnésium, ascorbyl phosphate de sodium (vendu par la Sté Roche sous la référence Stay-C 50) et ascorbyl glucoside (vendu par lan société Hayashibara). On peut également citer les actifs tels que le phloroglucinol et l'acide kojique.

Parmi les actifs hydrophiles sensibles à l'oxydation on préférera plus particulièrement l'acide ascorbique.

Des copolymères convenant plus particulièrement à la mise en oeuvre de l'invention sont des copolymères obtenu par copolymérisation d'une ou plusieurs unités anhydride maléique et dont les unités anhydride maléiques sont sous forme hydrolysée, et préférentiellement sous forme de sels alcalins, par exemple sous forme de sels d'ammonium, de sodium, de potassium ou de lithium.

Dans un aspect avantageux de l'invention, le copolymère possède une fraction molaire en unité anhydride maléique comprise entre 0,1 et 1, et plus préférentiellement entre 0,4 et 0,9.

Selon un aspect avantageux de l'invention le rapport molaire entre l'équivalent unité anhydride maléique et l'actif hydrophile sensible à l'oxydation varie entre 0,005 et 10 et préférentiellement entre 0,01 et 1.

La masse molaire moyenne en poids des copolymères d'anhydride maléique sera avantageusement comprise entre 1000 et 500 000 et de préférence entre 1000 et 50 000.

De façon préférentielle on utilisera un copolymère de styrène et d'anhydride maléique dans un rapport 50/50.

On pourra utiliser par exemple, le copolymère styrène/anhydride maléique (50/50), sous forme de sel d'ammonium à 30% dans l'eau vendu sous la référence SMA1000H® par la société ATOFINA ou le copolymère styrène/anhydride maléique (50/50), sous forme de sel de sodium à 40% dans l'eau vendu sous la référence SMA1000HNa® par la société ATOFINA.

Le copolymère est présent dans la composition selon l'invention en quantité suffisante pour obtenir l'effet recherché, c'est à dire en quantité suffisante pour stabiliser l'actif hydrophile sensible à l'oxydation. De préférence le copolymère est présent à une concentration comprise entre 0,1 et 40 % en poids, par rapport au poids total de la phase aqueuse, et plus particulièrement à une concentration comprise entre 0,1 et 10 % en poids, par rapport au poids total de la phase aqueuse.

Les compositions utilisées selon l'invention sont destinées à une application topique sur la peau et/ou ses phanères et contiennent donc un milieu physiologiquement acceptable, c'est-à-dire compatible avec les tissus cutanés tels que la peau, le cuir chevelu, les cils, les sourcils, les cheveux, les ongles et les muqueuses. Ce milieu physiologiquement acceptable peut être plus particulièrement constitué d'eau et éventuellement d'un solvant organique physiologiquement acceptable choisi par exemple parmi les alcools inférieurs comportant de 1 à 8 atomes de carbone et en particulier de 1 à 6 atomes de carbone, comme l'éthanol, l'isopropanol, le propanol, le butanol; les polyéthylène glycols ayant de 6 à 80 unités oxyde d'éthylène ; les polyols comme le propylène glycol, l'isoprène glycol, le butylène glycol, la glycérine, le sorbitol.

Quand le milieu physiologiquement acceptable est un milieu aqueux, il a généralement un pH compatible avec la peau, allant de préférence de 3 à 9 et mieux de 3,5 à 7,5.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique (liposomes, niosomes, oléosomes). Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle ; les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90$^R$ par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

Selon un autre mode de réalisation de l'invention, la composition utilisée contient en outre au moins un actif choisi parmi les agents desquamants capables d'agir, soit en favorisant l'exfoliation, soit sur les enzymes impliquées dans la desquamation ou la dégradation des cornéodesmosomes, les agents hydratants, les agents dépigmentants ou pro-pigmentants, les agents anti-glycation, les inhibiteurs de NO-synthase, les inhibiteurs de 5α-réductase, les inhibiteurs de lysyl et/ou prolyl hydroxylase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents anti-microbiens, les agents tenseurs, les agents anti-pollution ou anti-radicalaires, les agents anti-inflammatoires, les actifs lipolytiques ou ayant une activité favorable, directe ou indirecte, sur la diminution du tissu adipeux, les agents agissant sur la microcirculation, et les agents agissant sur le métabolisme énergétique des cellules.

De façon connue, la composition cosmétique ou dermatologique de l'invention peut contenir également des adjuvants habituels dans le domaine cosmétique ou dermatologique, tels que les gélifiants hydrophiles ou lipophiles, les conservateurs, les solvants, les parfums, les charges, les filtres UV, les bactéricides, les absorbeurs d'odeur, les matières colorantes, les extraits végétaux, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple, les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Selon un mode préféré de réalisation, les compositions conformes à l'invention peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés et choisis parmi les agents suivants, désignés ci-dessous sous leur nom INCI :

les dérivés de l'acide p-aminobenzoïque (PABA), en particulier le PABA, l'éthyl PABA, l'éthyl Dihydroxypropyl PABA, l'éthylhexyl Diméthyl PABA (vendu notamment sous le nom « ESCALOL 507 » par ISP), le glyceryl PABA, ou le PEG-25 PABA (vendu sous le nom « UVINUL P25 » par BASF),

les dérivés salicyliques, en particulier l'homosalate (vendu sous le nom « EUSOLEX HMS » par RONA/EM INDUSTRIES), l'éthylhexyl salicylate (vendu sous le nom « NEO HELIOPAN OS » par HAARMANN et REIMER), le dipropyleneglycol salicylate (vendu sous le nom « DIPSAL » par SCHER), ou le TEA salicylate (vendu sous le nom « NEO HELIOPAN TS » par HAARMANN et REIMER),

les dérivés de dibenzoylméthane, en particulier le butyl Methoxydibenzoylmethane (vendu notamment sous le nom commercial « PARSOL 1789 » par HOFFMANN LA ROCHE), ou l'isopropyl Dibenzoylmethane,

les dérivés cinnamiques, en particulier l'éthylhexyl Methoxycinnamate (vendu notamment sous le nom commercial « PARSOL MCX » par HOFFMANN LA ROCHE), l'isopropyl methoxy cinnamate, l'isoamyl Methoxy cinnamate (vendu sous le nom commercial « NEO HELIOPAN E 1000 » par HAARMANN et REIMER), le cinoxate, le DEA methoxycinnamate, le diisopropyl methylcinnamate, ou le glyceryl ethylhexanoate dimethoxycinnamate,

les dérivés de β,β-diphénylacryate, en particulier l'octocrylene (vendu notamment sous le nom commercial « UVINUL N539 » par BASF), ou l'étocrylene, (vendu notamment sous le nom commercial « UVINUL N35 » par BASF),

les dérivés de la benzophénone, en particulier la benzophenone-1 (vendue sous le nom commercial « UVINUL 400 » par BASF), la benzophenone-2 (vendue sous le nom commercial « UVINUL D50 » par BASF), la benzophenone-3 ou oxybenzone (vendue sous le nom commercial « UVINUL M40 » par BASF), la benzophenone-6 (vendue sous le nom commercial « HELISORB 11 » par NORQUAY), la benzophenone-8 (vendue sous le nom commercial « SPECTRA-SORB UV-24 » PAR AMERICAN CYANAMID), la benzophenone-12, ou le 2-(4-diéthylamino-2-hydroxybenzoyl)-benzoate de n-hexyle,

les dérivés du benzylidène camphre, en particulier le 3-benzylidene camphor (fabriqué sous le nom « MEXORYL SD» par CHIMEX), le 4-methylbenzylidene camphor (vendu sous le nom « EUSOLEX 6300 » par MERCK), ou le polyacrylamidomethyl benzylidene camphor (fabriqué sous le nom « MESORYL SW » par CHIMEX),

les dérivés de triazine, en particulier l'anisotriazine (vendue sous le nom commercial «TINOSORB S » par CIBA SPECIALTY CHEMICALS), l'éthylhexyl triazone (vendue notamment sous le nom commercial «UVINUL T150 » par BASF), la diethylhexyl butamido triazone (vendue sous le nom commercial « UVASORB HEB » par SIGMA 3V), ou la 2,4,6- tris-(4'amino-benzalmalonate de diisobutyle)-s-triazine,

les dérivés de benzotriazole, en particulier, le drometrizole trisiloxane (vendu sous le nom « SILATRIZOLE » par RHODIA CHIMIE), le methylène bis-benzotriazolyl tetramethylbutylphénol (vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS),

les dérivés anthraniliques, en particulier le menthyl anthranilate (vendu sous le nom commercial « NEO HELIOPAN MA » par HAARMANN et REIMER),

les dérivés d'imidazolines, en particulier l'éthylhexyl dimethoxybenzylidene dioxoimidazoline propionate,

les dérivés de benzalmalonate, en particulier le polyorganosiloxane à fonctions benzalmalonate (vendu sous la dénomination commerciale « PARSOL SLX » par HOFFMANN LA ROCHE),

et leurs mélanges,

les agents photoprotecteurs inorganiques choisis parmi les pigments ou bien encore les nanopigments (taille moyenne des particules primaires: généralement entre 5 nm et 100 nm, de préférence entre 10 nm et 50 nm) d'oxydes métalliques enrobés ou non comme par exemple les nanopigments d'oxyde de titane (amorphe ou cristallisé sous forme rutile et/ou anatase), de fer, de zinc, de zirconium ou de cérium qui sont tous des agents photoprotecteurs UV bien connus en soi ; les agents d'enrobage classiques tels que l'alumine et/ou le stéarate d'aluminium ; les nanopigments d'oxydes métalliques, enrobés ou non enrobés, sont en particulier décrits dans les demandes de brevets EP518772 et EP518773.

Les agents photoprotecteurs organiques plus particulièrement préférés sont choisis parmi l'éthylhexyl salicylate, l'éthylhexyl methoxycinnamate, l'octocrylene, la benzophenone-3, le 4-methylbenzylidene camphor, la 2,4,6-tris-(4'-amino benzalmalonate de diisobutyle)-s-triazine, l'anisotriazine, l'éthylhexyl triazone, la diethylhexyl butamido triazone, le méthylène bis-benzotriazolyl tetramethylbutylphénol, le drometrizole trisiloxane, et leurs mélanges.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

La composition selon l'invention peut être appliquée sur la peau, les poils, les cils, les cheveux, les ongles ou les lèvres, suivant l'usage auquel elle est destinée. Elle peut ainsi être utilisée dans un procédé de traitement cosmétique de la peau, comprenant l'application de la composition selon l'invention sur la peau.

En variante, la composition selon l'invention peut être utilisée pour la fabrication d'une préparation dermatologique.

Les exemples qui suivent servent à illustrer l'invention sans toutefois présenter un caractère limitatif. Les composés sont, selon le cas, cités en noms chimiques ou en noms CTFA (International Cosmetic Ingrédient Dictionary and Handbook).

**EXEMPLES**

**Exemple 1 : Test de conservation accéléré.**

Ce test a pour but d'étudier la dégradation d'un actif hydrophile sensible à l'oxydation après deux mois de conservation à 45°C. Diverses solutions ont été réalisées, et leurs compositions sont regroupées dans le tableau suivant :

Tableau I:

| Compositions (dans l'eau) | acide ascorbique | polymère 1 | polymère 2 |
|---|---|---|---|
| **solution A** (Témoin 1) | 15% | - | - |
| **solution B** | 15% | 1% - | |
| **solution C** | 15% | - | 1% |
| **solution D** (Témoin 2) | 5% | - | - |
| **solution E** | 5% | 1% | - |
| **solution F** | 5% | - | 1% |

Toutes les solutions sont ramenées à pH 6 avec KOH 8,9 mole/l
Les pourcentages des polymères sont donnés en matière active.

Polymère 1 : Copolymère styrène/anhydride maléique (50/50), sous forme de sel d'ammonium à 30% dans l'eau, vendu sous la référence SMA1000H® par la société ATOFINA.
Polymère 2 : Copolymère styrène/anhydride maléique (50/50), sous forme de sel de sodium vendu sous la référence SMA1000HNa® par la société ATOFINA.

Le taux de dégradation mesuré est donné par le rapport :

$$(C_0 - C_{2mois})/C_0$$

avec $C_0$ concentration en acide ascorbique à t=0 et $C_{2mois}$ la concentration en acide ascorbique à t=2 mois, dans les conditions indiquées dans le tableau ci-dessus.
La concentration en acide ascorbique est déterminée par la technique HPLC (système LaChrom Merck). Les conditions analytiques sont les suivantes :

Colonne Lichrosphere100 RP18 (250 mm)
Eluant : tampon phosphate O,1 M, pH 2,1
Débit : 1 ml/min.
Détection à 257 nm
Dilution de l'échantillon tel que la concentrattion en acide ascorbique soit comprise entre 0,05 et 1 mg/ml.

Les résultats obtenus sont regroupés dans le tableau II suivant :

<u>Tableau II :</u>

| | Taux de dégradation après 2 mois à 45°C (en %) | |
| --- | --- | --- |
| | sous air, flacon verre ambré | sous azote, flacon aluminium |
| solution A (Témoin 1) | 43 | 19,4 |
| solution B | 16 | 13,8 |
| solution C | 17,6 | 9,7 |
| solution D (Témoin 2) | 45,4 | 29,6 |
| solution E | 13,4 | 4,1 |
| solution F | 9 | 5,1 |

On constate d'après le tableau II que la stabilité de l'acide ascorbique, à une concentration de 5 ou 15%, est améliorée en présence des polymères 1 et 2 de l'invention, même en présence de l'oxygène de l'air, comparativement au témoin.

Les polymères cités étant hydrophiles, il suffira de les ajouter à une solution aqueuse d'acide ascorbique pour stabiliser ce dernier.

**Exemple 2 : Emulsion E/H**

| Phase A : | |
| --- | --- |
| Mélange de tartrate de di-alkyle ($C_{14-15}$ linéaire), alcool cétylstéarylique, alcool lauryque oxyéthyléné (25) oxypropyléné (25) (Cosmacol PSE) | 2,0 g |
| Glycéryl stéarate (and) PEG 100 stéarate | 2,7 g |
| Alcool stéarylique | 1,3 g |
| Cyclohexadiméthylsiloxane | 6,0 g |
| Isohexadecane | 2,0 g |
| Mélange de tocophérols naturels dans l'huile de soja (50/50) | 0,2 g |
| Phase B: | |
| Eau déminéralisée | 34,45 g |
| Disodium EDTA | 0,1 g |
| Conservateurs | 0,75 g |
| Ammonium polyacryloyldiméthyl taurate | 0,5 g |
| Phase C: | |
| Acide ascorbique | 5,0 g |
| Hydroxyde de potassium à 50% | 2,97 g |
| Eau déminéralisée | 38,69 g |
| Copolymère styrène/anhydride maléique, sel d'ammonium à 30% dans l'eau (SMA1000H® ATOFINA) | 3,34 g |

La composition ci-dessus est préparée de manière classique pour l'homme du métier, en émulsionnant la phase A dans la phase B à 80°C et en ajoutant la phase C à 35°C.
On obtient une crème douce à l'application avec une bonne stabilité de l'acide ascorbique.

**Exemple 3 : Emulsion E/H**

| **Phase A :** | |
|---|---|
| Acrylates/C$_{10-30}$ alkyl acrylate crosspolymer | 0,25 g |
| Xanthane | 0,4 g |
| Cyclohexadiméthylsiloxane | 10 g |
| **Phase B:** | |
| Eau déminéralisée | 21,63 g |
| Carbomer | 0,4 g |
| Diméthicone PEG-7 phosphate | 2,0 g |
| Hydroxyde de potassium à 50% | 0,32 g |
| **Phase C**: | |
| Acide ascorbique | 5,0 g |
| Copolymère styrène/anhydride maléique, sel de sodium à 40% dans l'eau (SMA1000HNa® ATOFINA) | 2,5 g |
| Hydroxyde de potassium à 50% | 2,97 g |
| Eau déminéralisée | 39,53 g |
| **Phase D**: | |
| Ethanol | 15,0 g |

La composition ci-dessus est préparée de manière classique pour l'homme du métier. Les phases A et B sont préparées à 60°C, et la phase C est préparée à température ambiante. La phase C est ajoutée à la phase B à 60°C, et la phase A est émulsionnée dans le mélange B+C. Après retour à température ambiante, la phase D est ajoutée.
On obtient une crème souple et fraîche à l'application avec une bonne stabilité de l'acide ascorbique.

**Exemple 4 : Emulsion E/H**

| **Phase A :** | |
|---|---|
| Eau déminéralisée | 59,68 g |
| Acide citrique | 0,05 g |
| Conservateurs | 0,95 g |
| Acide ascorbique | 5,0 g |
| Hydroxyde de potassium à 50% Copolymère styrène/anhydride maléique, sel d'ammonium à 30% dans l'eau | 2,98 g |
| (SMA1000H® ATOFINA) | 3,34 g |
| Biosaccharide Gum-1 | 2,0 g |
| Polysorbate 20 | 1,0 g |
| Citrate de sodium | 1,6 g |
| **Phase B :** | |
| Cyclopentasiloxane/diméthicone copolyol | 10,0 g |
| Cyclopentasiloxane | 8,0 g |
| Diméthicone / diméthiconol et vinyl diméthicone crosspolymer | 3,0 g |
| Mélange de tocophérols | 0,4 g |

(suite)

| Phase C : | |
|---|---|
| Polyacrylamide / C$_{13-14}$ isoparaffin / laureth-7 (SEPIGEL 305) | 2,0 g |

La composition ci-dessus est préparée de manière classique pour l'homme du métier. La phase A est préparée à température ambiante et émulsionnée lentement dans la phase B. La phase C est ensuite ajoutée au mélange.

On obtient une crème légère et douce à l'application avec une bonne stabilité de l'acide ascorbique.

**Exemple 5 : Microcapsules d'acide ascorbique**

On prépare une solution aqueuse d'acide ascorbique à 15% en poids, à pH 6, contenant 3,34 g de Copolymère styrène/anhydride maléique, sel d'ammonium à 30% dans l'eau (SMA1000H® ATOFINA). On émulsionne 5ml de cette solution dans 50 ml de chlorure de méthylène contenant 5% d'acéto-propionate de cellulose (CAP-482-0.5®, Eastman Chemical) à l'aide d'un homogénéiseur de type rotor-stator, pendant 5 min en maintenant la température en dessous de 25°C. Cette émulsion primaire est ensuite dispersée dans 500 ml d'une solution aqueuse contenant 1% d'alcool polyvinylique (Airvol 203®, AirProducts) et 7% de chlorure de sodium, à l'aide d'un disperseur Moritz pendant 20 min à température ambiante.

Le solvant de la suspension est ensuite évaporé à l'aide d'un évaporateur rotatif (Büchi B-480) pendant 5 heures à 40°C, à une pression de 75 kPa.

On obtient une dispersion de microcapsules dont la taille moyenne est de 30 µm avec un taux d'encapsulation de 75%, et un rendement de fabrication de 100%.

**Exemple 6 :Crème de jour contenant les microcapsules selon l'Exemple 5**

| Phase A | |
|---|---|
| Alcool cétylique | 4 g |
| Tristéarate de sorbitane | 0,9 g |
| Stéarate de polyéthylèneglycol | 2 g |
| Stéarate de glycérol | 3 g |
| Myristate de myristyle | 2 g |
| Palmitate d'octyle | 4,5 g |
| Parsol MCX ® (vendu par Hoffman-Laroche) | 3 g |
| Cyclopentasiloxane | 5 g |
| Conservateur | 0,1 g |
| **Phase B** | |
| Eau déminéralisée | 60,3 g |
| Conservateur | 0,15 g |
| Séquestrant | 0,05 g |
| **Phase C** | |
| Poudre de microcapsules selon l'exemple 5 | 15 g |

**Exemple 7 : Crème E/H :**

On prépare la composition suivante de manière classique pour l'homme du métier.

| Phase A | |
|---|---|
| Eau | 65,17 g |
| Phénoxyéthanol | 0,5 g |
| Méthylparaben | 0,3 g |

(suite)

| Phase A | |
|---|---|
| Glycérine | 3 g |
| Acide kojique | 1 g |
| Copolymère styrène/anhydride maléique, sel de sodium à 40% dans l'eau (SMA1000HNa® ATOFINA) | 0,63 g |
| Biosaccharide Gum-1 | 2g g |
| Polysorbate 20 | 1 g |
| Dipropylène glycol | 3 g |
| Miel | 1 g |
| **Phase B** | |
| Cyclopentasiloxane (and) diméthicone copolyol (Dow Corning® 5225C) | 10 g |
| Cyclopentasiloxane | 8 g |
| Tocophérol (and) glycine soja (soybean) oil | 0,4 g |
| Nylon-12 | 2 g |
| Polyacrylamide (and) C13-14 isoparaffin (and) laureth-7 (Sepigel305 SEPPIC) | 2 g |

**Exemple 8 : Gel EIH :**

On prépare la composition suivante de manière classique pour l'homme du métier.

| Phase A | |
|---|---|
| Cyclopentasiloxane (and) diméthicone copolyol | |
| (Dow Corning® 5225C) | 17,5 g |
| **Phase B** | |
| Sodium méthylparaben | 0,3 g |
| Chlorphenesin | 0,25 g |
| Eau | 81,07 g |
| Phloroglucinol | 0,5 g |
| Copolymère styrène/anhydride maléique, sel de sodium à 40% dans l'eau (SMA1000HNa® ATOFINA) | 0,38 g |

**Revendications**

1. Composition à usage topique contenant au moins un actif hydrophile sensible à l'oxydation et au moins un copolymère d'anhydride maléique et de styrène, dont les unités anhydride maléique sont sous forme hydrolysée, dans un milieu physiologiquement acceptable comprenant une phase aqueuse.

2. Composition selon l'une quelconque des revendications précédentes **caractérisée en ce que** l'actif hydrophile est choisi parmi l'acide ascorbique et ses dérivés tels que le 5,6-di-O-diméthylsilyl-ascorbate, le dl-alpha-tocopheryl-dl-ascorbyl-phosphate sel de potassium, l'ascorbyl phosphate de magnésium, l'ascorbyl phosphate de sodium, l'ascorbyl glucoside ; le phloroglucinol ; et l'acide kojique.

3. Composition selon l'une quelconque des revendications précédentes **caractérisée en ce que** l'actif hydrophile sensible à l'oxydation est l'acide ascorbique.

4. Composition selon l'une quelconque des revendications précédentes **caractérisée en ce que** les unités anhydride maléique hydrolysées sont sous forme de sels alcalins.

5. Composition selon l'une quelconque des revendications 1 à 3 **caractérisée en ce que** les unités anhydride maléique hydrolysées sont sous forme de sels choisis parmi les sels d'ammonium, de sodium, de potassium, de lithium.

6. Composition selon l'une quelconque des revendications précédentes **caractérisée en ce que** l'actif sensible à l'oxydation et le copolymère sont tous deux dans la phase aqueuse.

7. Composition selon l'une quelconque des revendications précédentes **caractérisée en ce que** le copolymère possède une fraction molaire en unité anhydride maléique comprise entre 0,1 et 1.

8. Composition selon l'une quelconque des revendications précédentes **caractérisée en ce que** le copolymère possède une fraction molaire en unité anhydride maléique comprise entre 0,4 et 0,9.

9. Composition selon l'une quelconque des revendications précédentes **caractérisée en ce que** le copolymère est un copolymère de styrène et d'anhydride maléique dans un rapport 50/50.

10. Composition selon l'une quelconque des revendications précédentes **caractérisée en ce que** le copolymère est un copolymère de styrène et d'anhydride maléique dans un rapport 50/50 sous forme de sel d'ammonium ou de sodium.

11. Composition selon l'une quelconque des revendications précédentes **caractérisée en ce que** le rapport molaire entre l'équivalent unité anhydride maléique et l'actif hydrophile sensible à l'oxydation varie entre 0,005 et 10.

12. Composition selon la revendication précédente **caractérisée en ce que** le rapport molaire entre l'équivalent unité anhydride maléique et l'actif hydrophile sensible à l'oxydation varie entre 0,01 et 1.

13. Composition selon l'une quelconque des revendications précédentes **caractérisée en ce que** le copolymère est présent à une concentration comprise entre 0,1 et 40 % en poids de la phase aqueuse.

14. Composition selon l'une quelconque des revendications précédentes **caractérisée en ce que** le copolymère est présent à une concentration comprise entre 0,1 et 10 % en poids de la phase aqueuse.

15. Utilisation d'un copolymère d'anhydride maléique et de styrène, dont les unités anhydride maléique sont sous forme hydrolysée, pour stabiliser un actif hydrophile sensible à l'oxydation.

16. Utilisation selon la revendication précédente **caractérisée en ce que** les unités anhydride maléique sous forme hydrolysées sont sous forme de sels alcalins.

17. Utilisation selon l'une des revendications 15 ou 16 **caractérisée en ce que** les unités anhydride maléique du copolymère sous forme hydrolysée sont sous forme de sels choisis parmi les sels d'ammonium, de sodium, de potassium, de lithium

18. Utilisation selon l'une quelconque des revendications 15 à 17 **caractérisée en ce que** le copolymère possède une fraction molaire en unité anhydride maléique comprise entre 0,1 et 1.

19. Utilisation selon l'une quelconque des revendications 15 à 18 **caractérisée en ce que** le copolymère est un copolymère de styrène et d'anhydride maléique dans un rapport 50/50 sous forme de sel d'ammonium ou de sodium.

20. Utilisation selon l'une quelconque des revendications 15 à 19 **caractérisée en ce que** l'actif hydrophile sensible à l'oxydation est l'acide ascorbique.

**Patentansprüche**

1. Zusammensetzung zur topischen Anwendung, die in einem physiologisch akzeptablen Medium, das eine wässerige Phase umfasst, mindestens einen oxidationsempfindlichen hydrophilen Wirkstoff und mindestens ein Copolymer von Maleinsäureanhydrid und Styrol anhält, dessen Maleinsäureanhydrideinheiten in hydrolysierter Form vorliegen.

2. Zusammensetzung nach einem der vorhergehenden Ansprüche, **dadurch gekennzeichnet, dass** der hydrophile Wirkstoff unter Ascorbinsäure und ihren Derivaten, beispielsweise 5,6-Di-O-dimethylsilylascorbat, dem Kaliumsalz von dl-alpha-Tocopheryl-dl-ascorbylphosphat, Magnesiumascorbylphosphat, Natriumascorbylphosphat, Ascorbylglucosid; Phloroglucin und Kojisäure ausgewählt ist.

3.  Zusammensetzung nach einem der vorhergehenden Ansprüche, **dadurch gekennzeichnet, dass** es sich bei dem oxidationsempfindlichen, hydrophilen Wirkstoff um Ascorbinsäure handelt.

4.  Zusammensetzung nach einem der vorhergehenden Ansprüche, **dadurch gekennzeichnet, dass** die hydrolysierten Maleinsäureanhydrideinheiten in Form von Alkalisalzen vorliegen.

5.  Zusammensetzung nach einem der Ansprüche 1 bis 3, **dadurch gekennzeichnet, dass** die hydrolysierten Maleinsäureanhydrideinheiten in Form von Salzen vorliegen, die unter den Ammoniumsalzen, Natriumsalzen, Kaliumsalzen und Lithiumsalzen ausgewählt sind.

6.  Zusammensetzung nach einem der vorhergehenden Ansprüche, **dadurch gekennzeichnet, dass** der oxidationsempfindliche Wirkstoff und das Copolymer beide in der wässerigen Phase enthalten sind.

7.  Zusammensetzung nach einem der vorhergehenden Ansprüche, **dadurch gekennzeichnet, dass** das Copolymer einen molaren Anteil der Maleinsäureanhydrideinheit im Bereich von 0,1 bis 1 aufweist.

8.  Zusammensetzung nach einem der vorhergehenden Ansprüche, **dadurch gekennzeichnet, dass** das Copolymer einen molaren Anteil der Maleinsäureanhydrideinheit im Bereich von 0,4 bis 0,9 aufweist.

9.  Zusammensetzung nach einem der vorhergehenden Ansprüche, **dadurch gekennzeichnet, dass** das Copolymer ein Copolymer von Styrol und Maleinsäureanhydrid in einem Verhältnis von 50/50 ist.

10. Zusammensetzung nach einem der vorhergehenden Ansprüche, **dadurch gekennzeichnet, dass** das Copolymer ein Copolymer von Styrol und Maleinsäureanhydrid in einem Verhältnis von 50/ 50 in Form des Ammoniumsalzes oder Natriumsalzes ist.

11. Zusammensetzung nach einem der vorhergehenden Ansprüche, **dadurch gekennzeichnet, dass** das Molverhältnis eines Äquivalents der Maleinsäureanhydrideinheit und des oxidationsempfindlichen hydrophilen Wirkstoffs im Bereich von 0,005 bis 10 liegt.

12. Zusammensetzung nach einem der vorhergehenden Ansprüche, **dadurch gekennzeichnet, dass** das Molverhältnis eines Äquivalents der Maleinsäureanhydrideinheit und des oxidationsempfindlichen hydrophilen Wirkstoffs im Bereich von 0,01 bis 1 liegt.

13. Zusammensetzung nach einem der vorhergehenden Ansprüche, **dadurch gekennzeichnet, dass** das Copolymer in einer Konzentration von 0,1 bis 40 Gew.-% der wässerigen Phase vorliegt.

14. Zusammensetzung nach einem der vorhergehenden Ansprüche, **dadurch gekennzeichnet, dass** das Copolymer in einer Konzentration von 0,1 bis 10 Gew.-% der wässerigen Phase enthalten ist.

15. Verwendung eines Copolymers von Maleinsäureanhydrid und Styrol, dessen Maleinsäureanhydrideinheiten in hydrolysierter Form vorliegen, zur Stabilisierung eines oxidationsempfindlichen hydrophilen Wirkstoffs.

16. Verwendung nach dem vorhergehenden Anspruch, **dadurch gekennzeichnet, dass** die in hydrolysierter Form vorliegenden Maleinsäureanhydrideinheiten als Alkalisalze vorliegen.

17. Verwendung nach einem der Ansprüche 15 oder 16, **dadurch gekennzeichnet, dass** die in hydrolysierter Form vorliegenden Maleinsäureanhydrideinheiten des Copolymers in Form von Salzen vorliegen, die unter den Ammoniumsalzen, Natriumsalzen, Kaliumsalzen und Lithiumsalzen ausgewählt sind.

18. Verwendung nach einem der Ansprüche 15 bis 17, **dadurch gekennzeichnet, dass** das Copolymer einen molaren Anteil der Maleinsäureanhydrideinheiten von 0,1 bis 1 aufweist.

19. Verwendung nach einem der Ansprüche 15 bis 18, **dadurch gekennzeichnet, dass** das Copolymer ein Copolymer von Styrol und Maleinsäureanhydrid in einem Verhältnis 50/50 in Form des Ammoniumsalzes oder Natriumsalzes ist.

20. Verwendung nach einem der Ansprüche 15 bis 19, **dadurch gekennzeichnet, dass** es sich bei dem oxidationsempfindlichen hydrophilen Wirkstoff um Ascorbinsäure handelt.

**Claims**

1. Composition for topical use comprising at least one oxidation-sensitive hydrophilic active principle and at least one copolymer of maleic anhydride and of styrene, the maleic anhydride units of which are in the hydrolysed form, in a physiologically acceptable medium comprising an aqueous phase.

2. Composition according to Claim 1, **characterized in that** the hydrophilic active principle is chosen from ascorbic acid and its derivatives, such as 5,6-di-O-dimethylsilylascorbate, the dl-$\alpha$-tocopheryl dl-ascorbyl phosphate potassium salt, magnesium ascorbyl phosphate, sodium ascorbyl phosphate or ascorbyl glucoside; phloroglucinol; and kojic acid.

3. Composition according to either one of the preceding claims, **characterized in that** the oxidation-sensitive hydrophilic active principle is ascorbic acid.

4. Composition according to any one of the preceding claims, **characterized in that** the hydrolysed maleic anhydride units are in the form of alkaline salts.

5. Composition according to any one of Claims 1 to 3, **characterized in that** the hydrolysed maleic anhydride units are in the form of salts chosen from ammonium, sodium, potassium or lithium salts.

6. Composition according to any one of the preceding claims, **characterized in that** the oxidation-sensitive active principle and the copolymer are both in the aqueous phase.

7. Composition according to any one of the preceding claims, **characterized in that** the copolymer has a molar fraction of maleic anhydride units of between 0.1 and 1.

8. Composition according to any one of the preceding claims, **characterized in that** the copolymer has a molar fraction of maleic anhydride units of between 0.4 and 0.9.

9. Composition according to any one of the preceding claims, **characterized in that** the copolymer is a copolymer of styrene and of maleic anhydride in a 50/50 ratio.

10. Composition according to any one of the preceding claims, **characterized in that** the copolymer is a copolymer of styrene and of maleic anhydride in a 50/50 ratio in the form of an ammonium or sodium salt.

11. Composition according to any one of the preceding claims, **characterized in that** the molar ratio of the maleic anhydride unit equivalent to the oxidation-sensitive hydrophilic active principle varies between 0.005 and 10.

12. Composition according to the preceding claim, **characterized in that** the molar ratio of the maleic anhydride unit equivalent to the oxidation-sensitive hydrophilic active principle varies between 0.01 and 1.

13. Composition according to any one of the preceding claims, **characterized in that** the copolymer is present at a concentration of between 0.1 and 40% by weight of the aqueous phase.

14. Composition according to any one of the preceding claims, **characterized in that** the copolymer is present at a concentration of between 0.1 and 10% by weight of the aqueous phase.

15. Use of a copolymer of maleic anhydride and of styrene, the maleic anhydride units of which are in the hydrolysed form, for stabilizing an oxidation-sensitive hydrophilic active principle.

16. Use according to the preceding claim, **characterized in that** the maleic anhydride units in the hydrolysed form are in the form of alkaline salts.

17. Use according to either of Claims 15 and 16, **characterized in that** the maleic anhydride units of the copolymer in the hydrolysed from are in the form of salts chosen from ammonium, sodium, potassium or lithium salts.

18. Use according to any one of Claims 15 to 17, **characterized in that** the copolymer has a molar fraction of maleic anhydride units of between 0.1 and 1.

19. Use according to any one of Claims 15 to 18, **characterized in that** the copolymer is a copolymer of styrene and of maleic anhydride in a 50/50 ratio in the form of an ammonium or sodium salt.

20. Use according to any one of Claims 15 to 19, **characterized in that** the oxidation-sensitive hydrophilic active principle is ascorbic acid.